(19) **Europäisches Patentamt / European Patent Office / Office européen des brevets**

(11) **EP 1 714 677 B1**

(12) **EUROPEAN PATENT SPECIFICATION**

(45) Date of publication and mention
of the grant of the patent:
**14.07.2010 Bulletin 2010/28**

(51) Int Cl.:
*A61Q 5/06* (2006.01)  *A61K 8/42* (2006.01)

(21) Application number: **05380081.9**

(22) Date of filing: **22.04.2005**

(54) **Long chain fatty acid dimethylaminopropylamide as levelling agent for dyeing kerationous fibres**

Dimethylaminopropylamid von langkettigen Fettsäuren als Ausgleichsmittel bei der Haarfärbung

Diméthylaminopropylamide d'acide gras long comme agent d'équilibrage pendant la teinture des cheveux

(84) Designated Contracting States:
**AT BE BG CH CY CZ DE DK EE ES FI FR GB GR HU IE IS IT LI LT LU MC NL PL PT RO SE SI SK TR**

(43) Date of publication of application:
**25.10.2006 Bulletin 2006/43**

(73) Proprietor: **KAO CORPORATION, S.A.**
**08210 Barbera del Valles,**
**(Barcelona) (ES)**

(72) Inventors:
• **Castán Barberán, Pilar**
**08210 Barberá del Vallès - Barcelona (ES)**
• **Abe, Hiroshi**
**08210 Barberá del Vallès - Barcelona (ES)**

(74) Representative: **Arias Sanz, Juan et al**
**ABG Patentes, S.L.**
**Avenida de Burgos, 16D**
**Edificio Euromor**
**28036 Madrid (ES)**

(56) References cited:
EP-A- 1 340 489  EP-A- 1 426 032
EP-A- 1 586 298  DD-A3- 271 219
DE-C1- 19 701 422

• R CANTERBERY PEPE, J A WENNINGER, G N MCEWEN: "International Cosmetic Ingredient Dictionary and Handbook, 9th ed." 2002, COSMETIC, TOILETRY, AND FRAGRANCE ASSOCIATION , WASHINGTON D.C. , XP002368835 * page 163, middle column - right-hand column * -& ANONYMOUS: "Incromine BB" CRODA USA - PERSONAL CARE PRODUCTS SEARCH RESULTS, [Online] 2006, pages 1-1, XP002368834 Retrieved from the Internet: URL: http://www.crodausa.com> [retrieved on 2006-02-20]
• DATABASE WPI Week 200480 Derwent Publications Ltd., London, GB; AN 2004-808781 XP002368839 "Cosmetic for preventing fading of hair during hair coloring" & JP 2004 323491 A (LION CORP) 18 November 2004 (2004-11-18)
• PATENT ABSTRACTS OF JAPAN vol. 018, no. 046 (C-1157), 25 January 1994 (1994-01-25) & JP 05 271035 A (SHISEIDO CO LTD), 19 October 1993 (1993-10-19)
• DATABASE WPI Week 200601 Derwent Publications Ltd., London, GB; AN 2006-005356 XP002368840 "Hair cosmetic" & JP 2005 343860 A (KAO CORP) 15 December 2005 (2005-12-15)
• DATABASE WPI Week 200550 Derwent Publications Ltd., London, GB; AN 2005-491923 XP002368841 "Hair cosmetic" & JP 2005 179197 A (LION CORP) 7 July 2005 (2005-07-07)
• ANONYMOUS: "Behenic acid dimethylaminopropylamide in hair dying compositions" RESEARCH DISCLOSURE, MASON PUBLICATIONS, HAMPSHIRE, GB, vol. 496, no. 13, August 2005 (2005-08), XP007135329 ISSN: 0374-4353

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

Remarks:
The file contains technical information submitted after the application was filed and not included in this specification

Remarks:
The file contains technical information submitted after the application was filed and not included in this specification

**Description**

**Technical field**

The present invention relates to dyeing compositions containing improved levelling agents.

**Prior Art**

It is known for centuries to dye keratinous fibres, for example human hair, with dyeing compositions comprising dyes or dye precursors.

Most hair colouring products fall under three major groupings:

1. Temporary hair colour
2. Semi permanent hair colour
3. Permanent hair colour.

Temporary hair colour is a leave on product that causes minimal damage to the hair. However, temporary hair colour causes stains, and leaches out under rain or with perspiration. Temporary hair colour washes out with the next shampoo. They are normally formulated with water-soluble direct dyes complexed with cationic surfactants to give a fine dispersion with particle sizes too great to penetrate through the cuticle into the cortex. As a result, the dye complex is deposited on the surface of the hair, from which it is easily removed by shampoo. Temporary hair colour also does not give any control to the consumer over the amount of colour deposited or the permanency of the colour supplied. Temporary hair colour does not result in a wide variety of colours and it has only a limited appeal.

Semi-permanent hair colour comes as a rinse, and it causes minimal damage to the hair. However, semi-permanent hair colour washes out to some degree with each shampoo and washes out completely within about 4 to 6 shampoos. Semi permanent hair colour employ low molecular weight dyes, which normally are simple derivatives of nitroanilines, nitrophenyldiamines, and nitroaminophenols, supplemented by a few azo and anthraquinone dyes. These dyes are of low enough molecular weight to penetrate into the cuticle and partially into the cortex of the hair. As a result, they are somewhat resistant to shampooing. Semi permanent hair colour does not give the consumer any control regarding the amount of colour deposited or the permanency of the colour.

Permanent hair colour generally comes in two parts: a dye solution and a developer solution. Permanent hair colour compositions do not contain dyestuffs in the conventional sense of the word. They contain colourless precursors which will react with an oxidizing agent inside the hair fibre to produce coloured molecules. Because of the damaging nature of conventional permanent dye treatments, most home permanent hair colouring products come with a post treatment conditioner. In a permanent hair colouring treatment, the dye solution and the developer solution, which contains an oxidizing agent (normally hydrogen peroxide), are mixed and then applied to the hair, which is then left for about 15 to about 35 minutes. The hair is then rinsed with water, treated with a post treatment conditioner, and then rinsed again with water.

The preparations for dyeing keratinous fibres often contain various reagents in addition to dyes. The purpose of these reagents is, in many cases, to control the manner in which dyes are adsorbed on the fibres and hence to control the evenness of the final dyeing, i.e. to produce a uniform distribution of the dyestuff.

These compounds, collectively referred to as "levelling agents", may be inorganic salts such as sodium sulphate, although various types of surfactants were found to be more effective. Anionic, cationic or amphoteric surfactants are commonly used, either alone or in blends, for dyeing both human and animal keratinous fibres. The mode of action of these levelling agents, especially the surfactants, usually involves formation of a complex with the dye. The complex is adsorbed more evenly by the fibres than the dye alone.

Improved evenness of dye adsorption is particularly important when dyeing damaged fibres in order to avoid an undesired appearance in the final dyed fibres, and thus surfactant-levelling agents are generally employed when dyeing damaged fibres. The damage to the fibres can be produced by repeated dyeing to such an extent that the tip and root portions of the fibres have markedly different dyeing properties. The above-mentioned undesired appearance refers to an undesired speckled effect arising from differences in colour between adjacent fibres or portions of the same, an effect often associated with damaged keratinous fibres.

It is desired to achieve the above-mentioned requirement of producing a uniform distribution of the dyestuff without losing the intensity of the hair colouring while obtaining optimal colour results (adequate colouring of some type of keratinous fibres).

Furthermore, a hair-dyeing product should be able to be confined upon application to the hair so that, on the one hand, it does not run over the face or outside the areas which it is desired to dye, and, on the other hand, a uniform and regular colouration may be obtained on the hair as a whole. Compositions comprising oxidation dye(s) prior to combining with an oxidizing agent should also be stable over time, such as, for example, from the rheological point of view.

Traditional thickeners, which can provide a gelling effect when diluted by water and/or surfactants, have been used conventionally to localize the dye product as applied on hair, so that the dye product does not touch the face or the area outside the area to be dyed. Such thickeners, chosen as appropriate, for example include crosslinked polyacrylic acid, hydroxyethylcelluloses, certain polyurethanes, waxes or mixtures of nonionic surfactants.

Commonly, fatty acid alkanolamides, optionally alkoxylated, like stearic acid mono- and diethanolamide (Stearamide MEA and Steramide DEA), coconut fatty acid mono- and diethanolamide (Cocamide MEA and Cocamide DEA), have been used in compositions for dyeing keratinous fibres, especially human hair as thickening agents or agents for adjusting the rheology.

DE-A-19701422 describes aqueous hair dyeing compositions with improved emulsion ability, consistency and colour intensity. Said aqueous hair dyeing compositions contain an oxidation dye precursor and 0.25-5 wt.% ethoxylated $C_{10}$-$C_{20}$ fatty acid alkanolamide. Preferably the hair colour compositions contain 0.5-2.5 wt.% PEG-5 Cocamide. It is stated that replacing PEG-5 Cocamide by PEG-3 Cocamide, PEG-7 Cocamide, PEG-3 Lauramide, PEG-6 Lauramide, PEG-9 Oleamide, PEG-4 Rapeseedamide and PEG-4 Stearamide similar improvements in the colour quality can be obtained.

WO-A-03053329 describes composition for dyeing hair, comprising, in a medium suitable for dyeing, at least an oxidation dye and more than 5 wt. % of the composition of at least an oxyethylene rapeseed fatty acid amide, preferably PEG-4 Rapeseedamide.

Other surfactants also used in hair dyeing compositions are polyoxyalkylenated carboxylic acid ethers (alkyl ether carboxylic acids) or salts thereof. Said compounds are described at least in the European patent applications EP-A-1279395, EP-A-1321133, EP-A-1321134, EP-A-1321135 and EP-A-1329216.

On the other hand, polyoxyethylene alkyl ether carboxylic acid monoethanolamides are also known in hair dyeing compositions, like C13-15 Pareth-2 Carboxamide MEA (Carboxylic $C_{13}$-$C_{15}$ alkyl ether acid monoethanolamide containing 2 moles of ethylene oxide), described in EP-A-1279395, EP-A-1321133, EP-A-1321134, EP-A-1321135 and EP-A-1329216 and in the International patent application WO-A-03072073.

The Japanese patent application JP-A-06256146 describes a cosmetic material which comprises

(i) at least one of amidoamine type surfactants of formula (1)

$$R_1-CO-NH-R_2-N(CH_2CH_3)_2$$

wherein $R_1$=fatty acid residue and $R_2$=alkyl, preferably stearic acid dimethylaminoethylamide; and
(ii) a guanidine derivative of formula (2)

$$R_3-[CO-NH-A_m]_n-NH-C(NH)_2$$

wherein $R_3$=alkyl or alkenyl; A=alkylene or alkenylene; M = 0 or 1 and n = 1-5 or its salts, and
(iii) a mono-N-long chain acyl basic amino acid lower alkyl ester salt
(iv) a tar type pigment.

US-A-4678475 describes a dye-conditioner composition that is non-staining to skin containing a certified violet dye (Ext. D & C Violet No. 2) and a dialkyl quaternary ammonium compound. Said composition could further comprises water, thickening agents, preservatives, emollients, one or more fatty alcohols and one or more fatty amido amines. Said fatty amido amines are used in order to increase the deposition of the violet dye into the hair. In the examples, stearamidopropyl dimethylamine is used.

Finally, US-B-6709468, US-B-6726729, US-B-6736861, and US-B-6770103 describe different methods for gradual permanent colouring of hair using compositions containing dyes and tertiary amidoamines (preferably stearamidopropyl dimethylamine) as conditioners.

EP-A1-1 340 489 describes compositions comprising levelling agent for dyeing keratinous fibres.

**Summary of the invention**

In the field of hair dyeing compositions, there exists a need for dyeing compositions containing improved levelling agents that make the colour of the dyed fibres more uniform, provide intense and chromatic shades of low selectivity and good fastness, while offering good stability of the compositions, good ease of application leading to uniform and regular colourations of the hair and a good cosmetic condition to the treated hair. It is thus the object underlying the present invention to provide dyeing compositions that are improved with respect to at least some of these properties.

The present invention thus provides dyeing compositions comprising

a) a dyestuff; and

b) fatty acid dimethylaminopropylamide and/or its salts, comprising at least 60 wt.% behenic acid dimethylaminopropylamide and/or its salts, the remainder being fatty acid dimethylaminopropylamide derived from other fatty acids, wherein the fatty acid dimethylaminopropylamide and/or its salts is present in an amount of 0.05 to 25 wt.% with respect to the total weight of the composition.

The present invention also provides a method for dyeing keratinous fibres with the dyeing compositions of the invention.

The present invention also provides the use of behenic acid dimethylaminopropylamide as levelling agent in compositions for dyeing of keratinous fibres, especially human hair.

**Description of the invention**

The dyeing composition of the present invention contain fatty acid dimethylaminopropylamide and/or its salts. At least 60 wt.% of the fatty acid dimethylaminopropylamide and/or its salts is behenic acid dimethylaminopropylamide and/or its salts, the remainder being fatty acid dimethylaminopropylamide derived from other fatty acids. According to the present invention, the term "fatty acid" defines a carboxylic acid having 12 to 28 carbon atoms, preferably 18 to 24 carbon atoms.

The fatty acid dimethylaminopropylamide to be used as component b) is generally obtained from the reaction of a behenic acid (docosanoic acid) composition and dimethylamino propyl amine.

Behenic acid dimethylaminopropylamide conforms to formula (I)

$$R\text{-}CO\text{-}NH\ (CH_2)_3\text{-}N(CH_3)_2 \qquad (I)$$

wherein R corresponds essentially to a $C_{21}$ alkyl group.

Behenic acid is preferably derived from natural fat and oil as well as synthetic triglycerides. Due to its possible natural origin, the fatty acid composition of behenic acid (i.e. the behenic acid composition) includes not only behenic acid, but also other fatty acids, such as small amounts of Palmitic Acid ($C_{16}$), Stearic Acid ($C_{18}$), Arachidic Acid ($C_{20}$), Lignoceric Acid ($C_{24}$) and others. Therefore, the content of behenic acid dimethylaminopropylamide, or a salt thereof, in the component b) (i.e. in the levelling agent) is 60 wt.% or higher, preferably equal or higher than 75 wt.%. It is particularly preferred that the content of $C_{21}$ in R is equal or higher than 85 wt.% and the content of $C_{17}$ is lower than 5 wt.%

According to the invention, the salts of the behenic acid dimethylaminopropylamide are obtained by neutralizing or partially neutralizing the behenic acid dimethylaminopropylamide with organic and/or inorganic acids, like hydrochloric acid, phosphoric acid, acetic acid, lactic acid, glycolic acid, malic acid, succinic acid, citric acid, L-glutamic acid, pyro-glutamic acid, $C_6$-$C_{22}$ fatty acids, like lauric acid, oleic acid, stearic acid and mixtures thereof, and alkyl ether carboxylic acids of formula

$$R\text{-}O\text{-}(CH_2CH_2O)_n\text{-}CH_2COOH$$

wherein R represents a $C_2$-$C_{10}$ alkyl chain, preferably $C_6$-$C_8$ alkyl, and n has a value in the range of 1 to 10, preferably 3-8.

The dyestuff can be a direct dye or a combination therefore. Examples of direct dyes include Acid Yellow 1 (C.I. 10316), Acid Yellow 3 (C.I. 47005), Acid Orange 7 (C.I. 15510), Acid Orange 87 (C.I. 45380:2), Acid Red 33 (C.I. 17200), Acid Violet 43 (C.I. 60730), Acid Blue 9 (C.I. 42090), Acid Green 25 (C.I. 61570), Acid Black 1 (C.I. 20470), Basic Blue 7 (C.I. 42595), Basic Blue 26 (C.I. 44045), Basic Blue 99 (C.I. 56059), Basic Violet 10 (C.I. 45170), Basic Violet 14 (C.I. 42515), Basic Brown 16 (C.I. 12250), Basic Brown 17 (C.I. 12251), Basic Red 2 (C.I. 50240), Basic Red 22 (C.I. 11055), Basic Red 51 (CAS RN 77061-58-9), Basic Red 76 (C.I. 12245), Basic Red 118 (C.I. 12251:1), Basic Yellow 57 (C.I. 12719), Basic Yellow 87 (CAS RN 68259-00-7), and Basic Orange 31 (CAS RN 97404-02-9)

If a single direct dye is used, it is preferably added in an amount of 0.01 to 20 wt.%, more preferably 0.05 to 10 wt. %, especially 0.1 to 5 wt.% on the basis of the entirety of the composition (after mixing of all the parts when a two-part or three-part composition is employed; this will apply equally hereinafter). When another direct dye is used in combination with the first one, the content of it in combination with the first direct dye preferably ranges from 0.05 to 10 wt.%, especially 0.1 to 5 wt.%, based on the whole composition.

The amount of behenic acid dimethylaminopropylamide to be added to compositions for dyeing keratinous fibres is between 0.05 wt.% to 25 wt.%, more preferably between 1 and 20 wt.%, even more preferably between 2.5 and 15 wt.% with respect to the total weight of the dyeing composition. In the case of two-part permanent hair dye compositions, the behenic acid dimethylaminopropylamide can be present in both parts. However, it is preferably present in the part containing the dyestuff, preferably in an amount of 0.1 to 30 wt.% and more preferably 2.5 to 20 wt.% with respect to the weight of said part of the composition.

The weight ratio of levelling agent (component b)) to dyestuff (component a)) is preferably in the range of 3:1 to 20:1, more preferably 4:1 to 15:1.

The hair dye composition of the present invention is preferably adjusted to pH 7 to 12, with pH 8 to 11 being more preferred. Buffering agents may be present in the compositions of the present invention in order to adjust the pH.

Suitable buffering agents are ammonium hydroxide, urea, ethylamine, dipropylamine, triethylamine and alkanediamines such as 1,3-diaminopropane, anhydrous alkaline alkanolamines such as, mono or di-ethanolamine, preferably those which are completely substituted on the amine group such as dimethylaminoethanol, polyalkylene polyamines such as diethylenetriamine or a heterocyclic amine such as morpholine as well as the hydroxides of alkali metals, such as sodium and potassium hydroxide, hydroxides of alkali earth metals, such as magnesium and calcium hydroxide, basic amino acids such as L-arginine, lysine, oxylysine and histidine and alkanolamines such as dimethylaminoethanol and aminoalkylpropanediol and mixtures thereof. Also suitable for use herein are compounds that form bicarbonate ($HCO_3^-$) by dissociation in water. Examples of suitable ion forming compounds are $Na_2CO_3$, $NaHCO_3$, $K_2CO_3$, $(NH_4)_2CO_3$, $NH_4HCO_3$, $CaCO_3$ and $Ca(HCO_3)_2$ and mixtures thereof.

Preferred for use as a buffering agent for the colouring compositions according to the present invention is ammonia, ammonium hydroxide and/or sodium hydroxide.

The buffering agent is preferably added in an amount of 0.01 to 20 wt.%, more preferably 0.1 to 10 wt.%, especially 0.5 to 5 wt.% based on the whole composition.

In the hair dye composition of the present invention, an oxidizing agent can be incorporated. In this case, hair dyeing and bleaching can be carried out simultaneously, which facilitates more vivid hair dyeing. Ordinarily employed oxidizing agents, for example, hydrogen peroxide, persulfates such as ammonium persulfate, potassium persulfate and sodium persulfate, perborates such as sodium perborate, percarbonates such as sodium percarbonate and bromates such as sodium bromate and potassium bromate are usable. Out of them, hydrogen peroxide is especially preferred. The oxidizing agent is added in an amount of 0.5 to 10 wt.%, especially 1 to 8 wt.%, on the basis of the entirety of the composition.

Since the stability of hydrogen peroxide solutions is influenced primarily by the temperature and the pH value; hydrogen peroxide is stable in the pH range from 2 to 5, it is necessary to use a buffering agent having a pH within this range. Dilute acids are suitable hydrogen peroxide buffering agents. Phosphoric acid is a preferred agent for buffering hydrogen peroxide solutions.

This pH adjustment can be effected by using well known acidifying agents in the field of treating keratinous fibers, and in particular human hair, such as inorganic and organic acids such as hydrochloric acid, tartaric acid, citric acid, phosphoric acid and carboxylic or sulphonic acids such as ascorbic acid, acetic acid, lactic acid, sulphuric acid, formic acid, ammonium sulphate and sodium dihydrogenphosphate/phosphoric acid, disodium hydrogen phosphate/ phosphoric acid, potassium chloride/hydrochloric acid, potassium dihydrogen phthalate/hydrochloric acid, sodium citrate/ hydrochloric acid, potassium dihydrogen citrate/hydrochloric acid, potassium dihydrogencitrate/citric acid, sodium citrate/ citric acid, sodium tartarate/tartaric acid, sodium lactate/lactic acid, sodium acetate/acetic acid, disodium hydrogenphosphate/citric acid and sodium chloride/glycine/hydrochloric acid and mixtures thereof.

Furthermore, the hair dye composition of the present invention may contain an oxidation dye. The incorporation of an oxidation dye enables markedly vivid dyeing not attainable by the single use of a direct dye. As the oxidizing agent, the above-exemplified oxidizing agents can be used, with hydrogen peroxide being particularly preferred. Alternatively, an oxidizing enzyme such as lactase can be employed. For the oxidation dye, known primary intermediates (developers) and couplers ordinarily employed for an oxidation type hair dye can be used.

The chemistry of oxidation dyeing involves a series of consecutive and competing reactions between hydrogen peroxide, primary intermediates (developers) and couplers. By appropriate selection of individual precursors, and adjustment of their absolute and relative concentrations, the formulator can produce a range of shades. By adjusting the ratio of peroxide to dye precursors, the colour of the hair can be changed from its natural tone to one which is lighter, darker, or similar in depth.

Examples of the developer include p-phenylenediamines having one or several groups selected from $NH_2$-, NHR- and NR2-groups (R represents a C(1-4) alkyl or hydroxyalkyl group) such as p-phenylenediamine, p-toluylenediamine, toluene-2,5-diamine, toluene-3,5-diamine, N-methyl-p-phenylenediamine, chloro-p-phenylenediamine, 2-(2'-hydroxyethylamino)-5-aminotoluene, N,N-bis-(2-hydroxyethyl)-p-phenylenediamine, 2-hydroxyethyl-p-phenylenediamine, 2,6-dimethyl-p-phenylenediamine, methoxy-p-phenylenediamine, 2,6-dichloro-p-phenylenediamine, 2-chloro-6-methyl-p-phenylenediamine, 6-methoxy-3-methyl-p-phenylenediamine, 2,5-diaminoanisole, N-(2-hydroxypropyl)-p-phenylenediamine and N-2-methoxyethyl-p-phenylenediamine; 2,5-diaminopyridine derivatives and 4,5-diaminopyrazole derivatives; 2,5-diaminotoluene; p-aminophenols such as p-aminophenol, 2-methyl-4-aminophenol, N-methyl-p-aminophenol, 3-methyl-4-aminophenol, 2,6-dimethyl-4-ami-nophenol, 3,5-dimethyl-4-aminophenol, 2,3-dimethyl-4-aminophenol and 2,5-dimethyl-4-aminophenol; o-aminophenols, o-phenylenediamines, 4,4'-diaminophenylamine and hydroxypropylbis (N-hydroxyethyl-p-phenylenediamine); and salts thereof.

Examples of the coupler include hydroquinone, 1 - naphthol, 1,5-dihydroxynaphthalene, 1,7-dihydroxynaphthalene, 2,7-dihydroxynaphthalene, 5-amino-2-methylphenol, 5-(2'-hydroxyethylamino)-2-methylphenol, 2,4-diaminoanisole, m-toluylenediamine, resorcinol, m-phenylenediamine, m-aminophenol, o-aminophenol, 4-chlororesorcinol, 2-methylresorcinol, 2,4-diaminophenoxyethanol, 2,6-diaminopyridine, 2-amino-3-hydroxypyridine, 4-hydroxyindole, 6-hydroxyindole, 2-hydroxybenzodioxane, 2,5-dihydroxypyridine, 2,4-diamino-6-hydroxypyrimidine, 2,4,6-triaminopyrimidine, 2-amino-4,6-dihydroxypyrimidine, 4-amino-2,6-dihydroxypyrimidine, 4,6-diamino-2-hydroxypyrimidine, 2,6-diaminopyridine, 4-methyl-5-aminophenol, 4-methyl-5-(2-hydroxyethyl)aminophenol, 6-hydroxybenzomorpholine, 3,3'-dihydroxydiphenylamine, and 1,3-bis(2,4-diaminophenoxy)propane; and salts thereof. As a developer or coupler, at least one of the above-exemplified ones can be used. Although no particular limitation is imposed on its content, it is added in an amount of 0.01 to 20 wt.%, especially 0.5 to 10 wt.% based on the whole composition.

The viscosity of the dye solution is preferably in the range of 20 to 50,000 mPas, whereas the viscosity of the developer solution is preferably in the range of 5 to 25,000 mPas. After mixing, the combined solution has preferably a viscosity in the range of 2,000 to 100,000 mPa·s, more preferably 15,000 to 100,000 mPa·s. The same viscosities are preferred for single-component hair dyeing compositions.

To the hair dye composition of the present invention, a known auto-oxidation dye typified by an indole or an indoline, or a known direct dye such as a nitro dye or a disperse dye can also be added. Addition of a polyol, polyol alkyl ether, cationic or amphoteric polymer or silicone to the hair dye composition of the present invention is preferred, because the resulting hair dye composition has improved cosmetic effects.

In addition to the above-described components, those ordinarily employed as a raw material for cosmetics can be added to the hair dye composition of the present invention, within an extent not impairing the advantages of the present invention. Examples of such an optional component include hydrocarbons, animal or vegetable fats and oils, higher fatty acids, organic solvents, penetration promoters, cationic surfactants, natural or synthetic polymers, higher alcohols, ethers, amphoteric surfactants, nonionic surfactants, protein derivatives, amino acids, antiseptics, chelating agents, stabilizing agents, antioxidants, plant extracts, crude drug extracts, vitamins, ceramides, pseudoceramides, colourants, perfumes and ultraviolet absorbers.

The composition for dyeing keratinous fibres of the present invention can be prepared in a conventional manner into a one-part composition, a two-part composition having a first-part component containing an alkali agent and a second-part component containing an oxidizing agent, or a third-part composition having, in addition to these two components, a powdery oxidizing agent such as persulfate. The direct dye can be incorporated in either one or both of these components of the two-part or three-part composition. The one-part type is applied to the hair directly, while the two-or three-part type is applied to the keratinous fibres after mixing these parts upon hair dyeing.

No particular limitation is imposed on the form of the composition for dyeing keratinous fibres of the present invention. Examples include powder, transparent liquid, emulsion, cream, mousse, gel, paste, aerosol, and aerosol foam. It preferably has a viscosity of 2,000 to 100,000 mPa·s in the stage of application to the hair (after mixing of all the parts when a two-part or three-part type composition is employed).

Temporary hair colouring compositions, semi permanent hair colouring compositions and permanent hair colouring compositions comprising behenic acid dimethylaminopropylamide and/or its salts are also included in the subject of the present invention.

A method for dyeing keratinous fibres, preferably human hair, comprising applying to such fibres, an effective amount of at least one composition of the present invention, is also included in the subject of the present invention.

The use of behenic acid dimethylaminopropylamide as levelling agent in compositions for dyeing keratinous fibres, preferably human hair, is also included in the subject of the present invention.

The following examples are given in order to provide a person skilled in the art with a sufficiently clear and complete explanation of the present invention.

## Examples

Preparation of the hair dyes compositions

In a manner known per se in the art, hair dye compositions as shown in Table 1 were prepared for the levelling agent according to the invention and for the comparative experiments.

The data appearing in each of the following tables represents weight percentage (wt.%).

Table 1

| 1st part (dye solution) | Components | Quantity |
|---|---|---|
| | Polyoxyethylene (13) Oleyl Ether | 10.00 |
| | Oleic Acid | 10.00 |
| | Levelling Agent | 5.00 |
| | Monoethanolamine | 7.50 |
| | Ethanol | 10.00 |
| | Toluene-2,5-diamine | 0.61 |
| | 1,3-Benzenediol (resorcinol) | 0.55 |
| | Sodium Sulfite | 0.50 |
| | Ascorbic Acid | 0.50 |
| | Tetrasodium ethylendiaminetetraacetate | 0.50 |
| | Deionized water | Balance |

| 2nd part (developer 35 wt. % solution) | Components | Quantity |
|---|---|---|
| | aqueous hydrogen peroxide | 16.29 |
| | Cetyl Alcohol | 2.00 |
| | Glycerin | 1.00 |
| | Dialkyldimethyl ammonium chloride | 1.00 |
| | Behentrimonium Chloride | 3.00 |
| | Phosphoric acid | Amount to adjust pH to 3.5 |
| | Deionized water | Balance |

Evaluation of the Levelling Effect

For the preparation of the damaged hair, yak tresses (white colour) were treated with a hydrogen peroxide solution.

An aqueous solution of hydrogen peroxide ($H_2O_2$) at 6 wt.% was prepared and adjusted at approx. pH 9 with ammonia. 700 mL of this solution were placed into a crystal vessel and was heated at 32°C. Then yak tresses were introduced in this solution for 30 min. After that they were removed and rinsed with hot tap water (40°C).

For each hair dye composition described before, having one part (dye solution) and second part (developer solution) were mixed at a weight ratio of 1:1 (the viscosity of said composition was measured with a Brookfield Viscometer LVD-VII+ (temperature = 20°C ; spindle= 4 ; speed 0.3 r.p.m.))

1 mL of the hair dye composition mixture was applied in each hair tress for 30 minutes at room temperature. 2 healthy yak tresses and 2 damaged yak tresses (treated with the hydrogen peroxide solution) were used. After that, each tress was rinsed with top water (1 min), washed with a standard shampoo (1 min), rinsed again with top water (1 min) treated with a standard hair conditioner (1 min, to detangle the hair tress), rinsed (1 min) again and dried over night at room temperature.

The intensity of the colour of hair tresses after the colouration treatment was determined with a spectrophotometer Spectraflash 600, supplied by DataColor (Illuminant: medium day light D65, 10° observer, %R SAV SCI UV Inc) using the CIE 1976-L*a*b* method.

The levelling effect (LE) of each levelling agent was calculated as follows:

```
LE = ΔE DH (colour intensity on damaged hair) - ΔE HH
(colour intensity on healthy hair)
```

Representing ΔE the difference in colour between each tress before and after the treatment with the dyeing composition (colour intensity)

Smaller values of LE indicate the better levelling effect of the levelling agent.

Results, which are averages of values for 2 samples, each of which underwent 10 separate measurements, are indicated in table 2.

Table 2

| Examples | Levelling Agent (INCI Name) | Viscosity after 1 min (mPa.s) | Levelling effect (LE) | Colour intensity (ΔE (HH)) |
|---|---|---|---|---|
| 1 | Behenamidopropyl dimethylamine | 35992 | 1.41 | 38.81 |
| C-1 | PEG-5 Cocamide | 19996 | 2.18 | 36.85 |
| C-2 | Cocamidopropyl dimethylamine | 19996 | 2.51 | 37.73 |
| C-3 | Stearamidopropyl dimethylamine | 25994 | 2.45 | 38.31 |

These results show clearly that the levelling agent in accordance with the invention (behenamidopropyl dimethylamine), exhibits not only higher colouring intensity on healthy hair (ΔE HH) but also better levelling effect (LE) than the chemicals having the greatest number of technical features in common with the invention (comparative experiments C2-C3).

Furthermore, the levelling agent in accordance with the invention exhibits also higher colouring intensity on healthy hair (ΔE HH) and better levelling effect (LE) in comparison with the levelling agents of the prior art (comparative experiment C1).

Finally, it is also remarked that the viscosity of the hair dye composition according to the invention, measured 1 minute after mixing the dye solution (first part) and the developer solution (second part), shows higher values than the comparative experiments.

From the above, it is clear that behenamidopropyl dimethylamine can act as thickening agent in hair dye compositions.

## Claims

1. A dyeing composition comprising

   a) a dyestuff; and
   b) fatty acid dimethylaminopropylamide and/or its salts, comprising at least 60 wt.% behenic acid dimethylaminopropylamide and/or its salts, the remainder being fatty acid dimethylaminopropylamide derived from other fatty acids.

   wherein the fatty acid dimethylaminopropylamide and/or its salts is present in an amount of 0.05 to 25 wt.% with respect to the total weight of the composition.

2. A composition according to claim 1 wherein the salts of the fatty acid dimethylaminopropylamide are obtained by neutralizing or partially neutralizing the fatty acid dimethylaminopropylamide with organic and/or inorganic acids.

3. A composition according to claim 1 or 2, wherein the salts of the fatty acid dimethylaminopropylamide are obtained by neutralizing or partially neutralizing the fatty acid dimethylaminopropylamide with an acid selected from the group consisting of hydrochloric acid, phosphoric acid, acetic acid, lactic acid, glycolic acid, malic acid, succinic acid, citric acid, L-glutamic acid, pyroglutamic acid, and $C_6$-$C_{22}$ fatty acids, and alkyl ether carboxylic acids of formula

   $$R-O-(CH_2CH_2O)_n-CH_2COOH$$

   wherein R represents a $C_2$-$C_{10}$ alkyl chain and n has a value in the range of 1 to 10.

4. A composition according to claims 1 to 3, further comprising an oxidizing agent.

5. A composition according to any of the preceding claims, further comprising an oxidation dye.

6. A composition according to any of the preceding claims which is a temporary, semi permanent, or permanent hair colouring composition.

7. A method for dyeing keratinous fibres comprising applying an effective amount of at least one composition according to any of claims 1 to 6 to keratinous fibres.

8. A method for dyeing keratinous fibres according to claim 7, wherein said keratinous fibres are human hair.

9. The use of behenic acid dimethylaminopropylamide and/or its salts as levelling agent in compositions for dyeing keratinous fibres..

10. The use according to claim 9, wherein said keratinous fibres are human hair.

**Patentansprüche**

1. Färbezusammensetzung, umfassend

   (a) einen Farbstoff; und
   (b) ein Fettsäure-dimethylaminopropylamid und/oder seine Salze, umfassend mindestens 60 Gew.% Behensäure-dimethylaminopropylamid und/oder seine Salze, wobei der Rest ein aus anderen Fettsäuren abgeleitetes Fettsäure-dimethylaminopropylamid ist,

   worin das Fettsäure-dimethylaminopropylamid und/oder seine Salze in einer Menge von 0,05 bis 25 Gew.%, bezogen auf das Gesamtgewicht der Zusammensetzung, vorliegt.

2. Zusammensetzung gemäss Anspruch 1, worin die Salze des Fettsäure-dimethylaminopropylamids durch Neutralisieren oder teilweises Neutralisieren des Fettsäure-dimethylaminopropylamids mit organischen und/oder anorganischen Säuren erhalten werden.

3. Zusammensetzung gemäss Anspruch 1 oder 2, worin die Salze des Fettsäure-dimethylaminopropylamids durch Neutralisieren oder teilweises Neutralisieren des Fettsäure-dimethylaminopropylamids mit einer Säure, die aus der Gruppe ausgewählt wird, die aus Salzsäure, Phosphorsäure, Essigsäure, Milchsäure, Glykolsäure, Äpfelsäure, Bernsteinsäure, Zitronensäure, L-Glutaminsäure, Pyroglutaminsäure und $C_{6-22}$-Fettsäuren besteht, und Alkylethercarbonsäuren der Formel

$$R\text{-}O\text{-}(CH_2CH_2O)_n\text{-}CH_2COOH$$

   erhalten werden, worin R eine $C_{2-10}$-Alkylkette darstellt und n einen Wert im Bereich von 1 bis 10 aufweist.

4. Zusammensetzung gemäss den Ansprüchen 1 bis 3, die ferner ein Oxidationsmittel umfasst.

5. Zusammensetzung gemäss mindestens einem der vorhergehenden Ansprüche, die ferner einen Oxidationsfarbstoff umfasst.

6. Zusammensetzung gemäss mindestens einem der vorhergehenden Ansprüche, die eine temporäre, semipermanente oder permanente Haarfärbezusammensetzung ist.

7. Verfahren zum Färben von keratinhaltigen Fasern, umfassend das Auftragen einer wirksamen Menge von mindestens einer Zusammensetzung gemäss mindestens einem der Ansprüche 1 bis 6 auf keratinhaltige Fasern.

8. Verfahren zum Färben von keratinhaltigen Fasern gemäss Anspruch 7, worin die keratinhaltigen Fasern menschliches Haar sind.

**9.** Verwendung von Behensäure-dimethylaminopropylamid und/oder seinen Salzen als Verlaufmittel in Zusammensetzungen zum Färben von keratinhaltigen Fasern.

**10.** Verwendung gemäss Anspruch 9, worin die keratinhaltigen Fasern menschliches Haar sind.

**Revendications**

**1.** Composition de teinture comprenant :

a) un colorant, et
b) du diméthylaminopropylamide d'acide gras et/ou ses sels, comprenant au moins 60% en poids de diméthylaminopropylamide d'acide béhénique et/ou ses sels, le restant étant du diméthylaminopropylamide d'acide gras dérivé d'autres acides gras,

**caractérisée en ce que** le diméthylaminopropylamide d'acide gras et/ou ses sels est présent dans une proportion de 0,05 à 25% en poids par rapport au poids total de la composition.

**2.** Composition selon la revendication 1, **caractérisée en ce que** les sels du diméthylaminopropylamide d'acide gras sont obtenus en neutralisant totalement ou partiellement le diméthylaminopropylamide d'acide gras avec des acides organiques et/ou inorganiques.

**3.** Composition selon la revendication 1 ou 2, **caractérisée en ce que** les sels du diméthylaminopropylamide d'acide gras sont obtenus en neutralisant totalement ou partiellement le diméthylaminopropylamide d'acide gras avec un acide choisi dans le groupe composé de l'acide chlorhydrique, l'acide phosphorique, l'acide acétique, l'acide lactique, l'acide glycolique, l'acide malique, l'acide succinique, l'acide citrique, l'acide L-glutamique, l'acide pyroglutamique, et les acides gras $C_6$-$C_{12}$, et les acides carboxyliques d'éther d'alkyl de formule :

$$R-O-(CH_2CH_2O)_n-CH_2COOH$$

**caractérisée en ce que** R représente une chaîne d'alkyl $C_2$-$C_{10}$ et n a une valeur comprise entre 1 et 10.

**4.** Composition selon les revendications 1 à 3, comprenant de plus un agent oxydant.

**5.** Composition selon l'une quelconque des revendications précédentes, comprenant de plus une teinture d'oxydation.

**6.** Composition selon l'une quelconque des revendications précédentes qui est une composition de coloration capillaire temporaire, semi-permanente ou permanente.

**7.** Méthode de coloration des fibres de kératine comprenant l'application d'une quantité efficace d'au moins une composition selon l'une quelconque des revendications 1 à 6 sur les fibres de kératine.

**8.** Méthode de coloration des fibres de kératine selon la revendication 7, **caractérisée en ce que** les dites fibres de kératine sont des cheveux humains.

**9.** Utilisation du diméthylaminopropylamide d'acide béhénique et/ou ses sels comme agent de stabilisation dans des compositions pour la coloration de fibres de kératine.

**10.** Utilisation selon la revendication 9, **caractérisé en ce que** les dites fibres de kératine sont des cheveux humains.

## REFERENCES CITED IN THE DESCRIPTION

**Patent documents cited in the description**

- DE 19701422 A
- WO 03053329 A
- EP 1279395 A
- EP 1321133 A
- EP 1321134 A
- EP 1321135 A
- EP 1329216 A
- WO 03072073 A

- JP 06256146 A
- US 4678475 A
- US 6709468 B
- US 6726729 B
- US 6736861 B
- US 6770103 B
- EP 1340489 A1